# FASCICULE DE BREVET EUROPEEN

(11) **EP 0 905 113 B1**
(45) Date de publication et mention de la délivrance du brevet: **31.07.2002**
(21) Numéro de dépôt: 98203169.2
(22) Date de dépôt: 18.09.1998
(51) Int. Cl.: C07C 17/08, C07C 21/04

(54) **Procédé de préparation de 2-chloroprop-1-ène**
Verfahren zur Herstellung von 2-Chlorprop-1-en
Process for the preparation of 2-chloroprop-1-ene

(30) Priorité: 24.09.1997 FR 9711976
(43) Date de publication de la demande: 31.03.1999
(73) Titulaire: SOLVAY (Société Anonyme), 1050 Bruxelles (BE)
(72) Inventeur: Schoebrechts, Jean-Paul, 5980 Grez-Doiceau (BE); Janssens, Francine, 1800 Vilvoorde (BE)
(74) Mandataire: Jacques, Philippe

(56) Documents cités:
- EP-A- 0 132 544
- BE-A- 701 874
- US-A- 3 420 902
- CHEMICAL ABSTRACTS, vol. 116, no. 5, 3 février 1992 Columbus, Ohio, US; abstract no. 040896, KAWAMURA Y ET AL: "Preparation of 2-halo-1-alkene by reaction of allene derivative or 1-alkyne with hydrogen halide" XP000250893 & JP 03 206053 A (IDEMITSU PETROCHEMICAL CO., LTD.;JAPAN) 9 septembre 1991

## Description

La présente invention concerne un procédé de préparation du 2-chloroprop-1-ène.

Le 2-chloroprop-1-ène est un intermédiaire dans la synthèse du 1,1,1,3,3-pentachlorobutane, lui-même précurseur du dérivé fluoré correspondant connu sous l'abréviation HFC-365mfc utilisé comme solvant et comme agent gonflant dans la préparation de mousses cellulaires polymériques.

Il est connu de former du 2-chloroprop-1-ène au départ d'un mélange de composés comprenant notamment du méthylacétylène et du propadiène, par hydrochloration en phase gazeuse en présence d'un catalyseur supporté (brevet FR 1.505.099, demande de brevet japonais JP 03/206.053). La stabilité de tels catalyseurs d'hydrochloration utilisés en phase gazeuse est toutefois insuffisante pour une exploitation industrielle aisée. La toxicité de certains des catalyseurs mis en oeuvre et leur action néfaste sur l'environnement constituent en outre des inconvénients importants.

Le brevet US 4.480.121 décrit un tel procédé, réalisé en présence de vapeur d'eau à température élevée. Il est cependant bien connu qu'en présence d'eau le chlorure d'hydrogène constitue un milieu particulièrement corrosif.

En conséquence, la présente invention a pour but de fournir un procédé alternatif de préparation du 2-chloroprop-1-ène au départ de méthylacétylène ou de propadiène, dans lequel le 2-chloroprop-1-ène est obtenu avec d'excellents rendements et qui ne présente pas les inconvénients des procédés antérieurs.

L'invention concerne donc un procédé de préparation du 2-chloroprop-1-ène par réaction de méthylacétylène et/ou de propadiène avec du chlorure d'hydrogène dans un milieu liquide renfermant au moins
(a) un catalyseur d'hydrochloration qui comprend au moins un composé choisi parmi les composés des métaux du groupe VIIIa et des lanthanides; et
(b) un solvant organique capable de solubiliser le catalyseur.

De manière avantageuse, le procédé selon l'invention se déroule en absence substantielle d'eau.

Comme solvant capable de solubiliser le catalyseur, on peut utiliser notamment un nitrile, un composé organique du phosphore ou un sel d'ammonium et leurs mélanges.

Sont notamment utilisables comme solvant dans le procédé selon l'invention les nitriles aliphatiques de formule générale CH₃-(CH₂)ₙ-CN avec n un entier de 3 à 7; les dinitriles aliphatiques de formule générale NC-(CH₂)ₘ-CN avec m un entier de 3 à 6; des nitriles aromatiques tels que le benzonitrile et le toluonitrile; le phosphite de triéthyle, le phosphite de tributyle, le phosphite de triphényle, le phosphate de tributyle; et, de préférence, les sels d'ammonium répondant à la formule générale 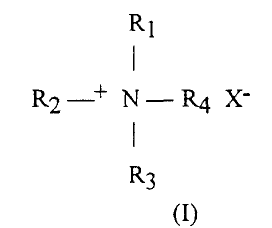 dans laquelle R₁, R₂, R₃ et R₄ représentent des atomes d'hydrogène, des groupements alkyle ou aryle, identiques ou différents, l'un d'eux au moins étant un groupement alkyle ou aryle et X représente un anion, de préférence l'anion chlorure. Eventuellement, R₁ et R₃ peuvent former ensemble au moyen d'atomes de carbone les reliant un cycle, par exemple à 5 ou à 6 atomes de carbone, lesquels peuvent être substitués par des groupements alkyle. Par groupement alkyle, on entend toute chaîne carbonée linéaire ou ramifiée, éventuellement substituée par un ou plusieurs groupements aryle. Par groupement aryle, on entend tout radical aromatique éventuellement substitué par un ou plusieurs groupements alkyle. Le nombre total d'atomes de carbone dans ce composé aminé est avantageusement au moins égal à 8. Il est de préférence au moins égal à 12. Le nombre d'atomes de carbone dans ce composé est généralement au plus égal à 40. Il est de préférence au plus égal à 30.

Par sel d'ammonium, on entend désigner un ou plusieurs sels d'amine, y compris tout mélange de sels de plusieurs amines, par exemple, plusieurs composés isomériques. Un tel mélange de sels de plusieurs amines peut aussi être mis en oeuvre, notamment en raison de sa grande disponibilité ou de son plus faible coût par rapport aux composés purs. Un exemple d'un tel sel d'ammonium comprenant un mélange de divers composés répondant à la formule (I) est obtenu par la réaction du chlorure d'hydrogène avec des produits commerciaux tels que les amines primaires tert-alkyle PRIMENE® 81-R et PRIMENE® JM-T de ROHM AND HAAS CO, constituées de mélanges d'amines isomériques respectivement en C₁₂-C₁₄ et en C₁₈-C₂₂. Dans certains cas, il peut aussi s'avérer intéressant de mélanger volontairement des chlorures de différentes amines en raison de l'existence d'eutectiques entre ces composés, présentant un point de fusion inférieur à celui de chacun des constituants.

Une préférence particulière est montrée pour les sels d'ammonium liquides à la température ambiante. Une préférence toute particulière est montrée pour les chlorures d'ammonium liquides à la température ambiante. D'une manière générale, les amines stériquement encombrées ou celles possédant au moins 2 groupes en C₆-C₂₀ forment des chlorures liquides à la température ambiante.

Des sels d'ammonium liquides à température ambiante sont ceux où au moins un des 4 substituants de l'atome d'azote représente un groupe alkyle possédant une longue chaîne carbonée ramifiée et dont le nombre d'atomes de carbone dans le composé est au moins égal à 8, de préférence au moins égal à 12. D'autres sels d'ammonium liquides à la température ambiante sont ceux où au moins 2 substituants de l'atome d'azote sont des groupes alkyle en C₆-C₂₀.

Des sels d'ammonium utilisables dans le procédé selon l'invention sont notamment les chlorhydrates d'amine c'est-à-dire des composés de formule I dans laquelle R₄ représente de l'hydrogène et X est un ion chlorure. Le chlorure de méthyltrioctylammonium est un autre sel d'ammonium préféré.

Le catalyseur d'hydrochloration utilisé dans le procédé de la présente invention comprend au moins un composé de métal du groupe VIIIa ou des lanthanides (selon la version IUPAC de 1970 du tableau périodique des éléments). Avantageusement, les composés des métaux mis en oeuvre sont choisis parmi les halogénures. Une préférence est montrée pour les chlorures ou les bromures mais tout autre composé pouvant se transformer en chlorure en présence de chlorure d'hydrogène peut aussi être utilisé. Peuvent également être mis en oeuvre, les composés de métal du groupe VIIIa complexés par des systèmes riches en électrons tels que les amines, les composés oxygénés comme les composés carbonylés ou les éthers, cycliques ou acycliques, les composés soufrés, les composés aromatiques ou les composés porteurs de noyaux aromatiques. Sont avantageusement considérés comme composés de métal utilisables, les sels formés entre un métal du groupe VIIIa et un composé organique acide, non seulement avec les acides carboxyliques mais aussi avec d'autres composés, tels que l'acétylacétone. On peut également mettre en oeuvre comme catalyseur des complexes de métaux du groupe VIIIa dans lequel le métal a la valence 0 tels que les complexes formés avec la triphénylphosphine ou l'oxyde de triphénylphosphine.

Les composés de métaux des lanthanides utilisables dans la présente invention sont généralement choisis parmi les composés de cérium, de praséodyme ou de néodyme ou leurs mélanges. Les composés de cérium sont préférés. Le chlorure de cérium est particulièrement préféré.

De préférence, le catalyseur comprend au moins un composé de métal du groupe VIIIa. Les composés de métaux du groupe VIIIa utilisables dans la présente invention sont généralement choisis parmi les composés de fer, de cobalt, de nickel, de ruthénium, de rhodium, de palladium, d'osmium, d'iridium, de platine ou leurs mélanges.

Avantageusement, le composé de métal du groupe VIIIa mis en oeuvre est choisi parmi les composés de platine, de rhodium ou de palladium. Les composés de platine sont tout particulièrement préférés.

Comme composés de platine, on peut utiliser un halogénure comme, par exemple, le chlorure de platine (II), le bromure de platine (II) et l'iodure de platine (II) ou un platinochlorure de métal alcalin ou de métal alcalino-terreux tel que le Na₂PtCl₄; on peut aussi utiliser l'acide hexachloroplatinique (H₂PtCl₆), le Na₂PtCl₆, le K₂PtCl₆, le (NH₄)₂PtCl₄ ou l'acétylacétonate de platine (II) L'acétylacétonate de platine (II) et les halogénures de platine sont préférés et parmi ces derniers, le chlorure de platine (II) et le bromure de platine (II) sont particulièrement préférés.

Avantageusement, la nature et/ou la quantité de catalyseur mise en oeuvre est telle que, tout le catalyseur se trouve sous forme dissoute dans le solvant organique. On peut cependant aussi mettre en oeuvre un catalyseur en quantité ou de nature telle qu'une fraction au moins de celui-ci soit présente dans le milieu liquide sous forme solide dispersé, sans porter préjudice à l'invention. La quantité de catalyseur engagé est généralement supérieure ou égale à 1 millimole par litre de milieu liquide. De préférence, elle est supérieure ou égale à 10 millimoles par litre de milieu liquide. Avantageusement, elle est supérieure ou égale à 20 millimoles par litre de milieu liquide. La quantité de catalyseur est habituellement inférieure ou égale à 200 millimoles par litre de milieu liquide. De préférence, elle est inférieure ou égale à 150 millimoles par litre de milieu liquide. Avantageusement, elle est inférieur ou égale à 100 millimoles par litre de milieu liquide.

Dans un mode de réalisation préféré du procédé selon l'invention, on met aussi en oeuvre un cocatalyseur, lequel comprend au moins un composé d'au moins un métal des groupes Ib ou IVb tel que le cuivre, l'argent, l'étain ou le plomb. Une préférence est marquée pour des métaux tels que le cuivre et l'étain, en particulier le cuivre. De préférence, le composé de métal des groupes Ib ou IVb mis en oeuvre comme cocatalyseur dans ce mode de réalisation est un chlorure. Une préférence particulière est montrée pour le chlorure de cuivre (II), tout particulièrement lorsque le catalyseur est un halogénure de platine (II). Généralement, le cocatalyseur est mis en oeuvre dans un rapport molaire par rapport au catalyseur supérieur à 0,1. De préférence, ce rapport molaire est supérieur ou égal à 1. Avantageusement, ce rapport molaire est supérieur ou égal à 2. Toutefois, ce rapport molaire est habituellement inférieur à 20. De préférence, ce rapport molaire est inférieur ou égal à 15. Avantageusement, ce rapport molaire est inférieur ou égal à 10. Le cocatalyseur peut être introduit au début de la réaction, en même temps que le catalyseur, ou il peut être introduit en cours de réaction.

De manière avantageuse, outre le catalyseur, le solvant et l'éventuel cocatalyseur, le milieu liquide comprend au moins un co-solvant organique. Le choix de la nature du co-solvant organique mis en oeuvre est conditionné notamment par la nécessité qu'il soit inerte vis-à-vis des réactifs dans les conditions de réaction, qu'il soit miscible avec le solvant à la température de réaction et qu'il soit capable de le solubiliser, en particulier lorsque ce dernier est solide à température ambiante. Par ailleurs, pour des raisons de sécurité et de facilité d'emploi, on donne la préférence aux co-solvants organiques peu volatils. Le choix du co-solvant organique est aussi influencé par sa capacité de dissolution du méthylacétylène et/ou du propadiène. Des co-solvants satisfaisant aux différents critères exposés ci-dessus sont choisis parmi les hydrocarbures aliphatiques, cycloaliphatiques et aromatiques et leurs mélanges, par exemple les paraffines en C₇ à C₁₅ et les alkylbenzènes, notamment les xylènes, les propylbenzènes, les butylbenzènes, les méthyléthylbenzènes. Le co-solvant mis en oeuvre est de préférence choisi parmi les produits commerciaux constitués de mélanges d'hydrocarbures aliphatiques tels que le produit ISOPAR® de ESSO ou le produit SHELLSOL® D70 de SHELL ou de mélanges de composés aromatiques tels que le produit SOLVESSO® de ESSO ou le produit SHELLSOL® AB de SHELL.

Des co-solvants ayant donné de bons résultats sont les co-solvants aliphatiques saturés tels que le produit SHELLSOL® D70, constitué de coupes pétrolières ayant un point d'ébullition supérieur ou égal à environ 190 °C et inférieur ou égal à environ 250 °C.

D'autres co-solvants envisageables sur base des divers critères donnés ci-dessus sont certains composés halogénés lourds, tels que des halogénoalcanes, halogénobenzènes et autres dérivés halogénés de composés aromatiques.

Il est souvent avantageux de mettre en oeuvre un solvant et un co-solvant, notamment lorsque le milieu liquide est trop visqueux pour permettre une parfaite dispersion des réactifs dans le solvant. Dans ce cas, le rapport volumique entre le co-solvant et le solvant est généralement supérieur ou égal à 0,01. De préférence, ce rapport est supérieur ou égal à 0,10. Avantageusement, ce rapport est supérieur ou égal à 0,25. Généralement, ce rapport est inférieur ou égal à environ 100. De préférence, il est inférieur ou égal à environ 10. Avantageusement, ce rapport est inférieur ou égal à 5.

Dans le procédé selon l'invention, le milieu liquide le plus particulièrement préféré contient du bromure de platine (II) ou du chlorure de platine (II) comme catalyseur, un chlorure d'ammonium de formule (I), liquide à température ambiante comme solvant, du chlorure de cuivre (II) comme cocatalyseur et un mélange d'hydrocarbures aliphatiques saturés comme co-solvant.

Le procédé de fabrication du 2-chloroprop-1-ène selon l'invention est réalisé par mise en contact de méthylacétylène et/ou de propadiène avec du chlorure d'hydrogène dans tout réacteur approprié renfermant le milieu liquide. Dans le procédé selon l'invention, on préfère utiliser comme réactif un mélange d'hydrocarbures contenant du méthylacétylène et du propadiène, par exemple celui commercialisé par Air Liquide sous le nom de TETRENE®. Sa composition molaire est environ de 25 % de méthylacétylène, 13 % de propadiène, 46 % de propylène, 4 % de propane et 12 % d'hydrocarbures en C4. De préférence, le milieu liquide est saturé en chlorure d'hydrogène avant d'introduire le métylacétylène et/ou le propadiène dans le réacteur.

Le procédé selon l'invention peut être réalisé, de façon discontinue ou de façon continue, classiquement dans tout appareillage favorisant l'échange gaz-liquide telle qu'une colonne à plateaux, une colonne noyée à empilages ou une colonne à bulles. Avantageusement, on ajuste le débit des gaz introduits au réacteur de manière à maximaliser la surface d'échange gaz/liquide.

Dans le procédé selon l'invention, le rapport molaire entre le chlorure d'hydrogène et le méthylacétylène et/ou le propadiène introduits dans le réacteur est en général supérieur ou égal à environ 0,5. De préférence, ce rapport est supérieur ou égal à 1. En général, ce rapport molaire est inférieur ou égal à environ 10. De préférence, ce rapport est inférieur ou égal à 5. De bons résultats ont été obtenus avec un rapport molaire entre le chlorure d'hydrogène et le méthylacétylène et/ou le propadiène introduits dans le réacteur inférieur ou égal à environ 2,5. Le méthylacétylène et/ou le propadiène et le chlorure d'hydrogène peuvent être mis en contact dans le réacteur ou mélangés préalablement à leur introduction dans le réacteur.

Le procédé de l'invention est réalisable de la température ambiante jusqu'à environ 200 °C. A plus haute température, le catalyseur a tendance à se dégrader. La température de réaction préférée, c'est-à-dire celle offrant le meilleur compromis entre productivité, rendement et stabilité du catalyseur est supérieure ou égale à 80 °C. Les meilleurs résultats sont obtenus à des températures supérieures ou égales à environ 100 °C. De préférence, la température de réaction ne dépasse pas environ 180 °C. Une température de réaction inférieure ou égale à environ 160 °C est particulièrement préférée.

La pression est généralement supérieure ou égale à la pression atmosphérique et égale ou inférieure à 15 bars. De préférence, la pression est inférieure ou égale à 10 bars. Une préférence particulière est montrée pour une pression inférieure ou égale à 5 bars. Avantageusement, le procédé de l'invention se déroule de façon continue à la pression de 1 bar ou à une pression proche de 1 bar. Le débit des réactifs, généralement gazeux, doit être suffisant pour permettre un brassage efficace du milieu liquide, à moins que celui-ci soit agité mécaniquement.

Dans un procédé en continu, le temps de séjour, qui est le rapport entre le volume de milieu liquide dans le réacteur et le débit volumique des réactifs, est généralement supérieur ou égal à 0,5 seconde. Avantageusement, le temps de séjour est supérieur ou égal à 1 seconde. En général, le temps de séjour ne dépasse pas 5 minutes. Il est le plus souvent inférieur ou égal à 2 minutes. Avantageusement, il est inférieur ou égal à 1 minute.

Dans le but d'augmenter la quantité de méthylacétylène et/ou de propadiène dissous dans le milieu liquide, il est également possible, lorsque le solvant mis en oeuvre est un chlorhydrate d'amine, de mettre en oeuvre le procédé selon l'invention de telle sorte que seul le méthylacétylène et/ou le propadiène est introduit dans le réacteur sous forme gazeuse, où il réagit avec le chlorure d'hydrogène présent dans la phase liquide sous forme de chlorhydrate d'amine, celui-ci étant régénéré par mise en contact d'une navette liquide renfermant l'amine avec du chlorure d'hydrogène en dehors du réacteur.

L'invention est illustrée dans les exemples suivants.

### Exemple 1

La réaction a été conduite dans une colonne à bulle en pyrex munie d'un double enveloppe dans laquelle circule de l'huile thermostatisée à la température de l'essai et surmontée d'un réfrigérant refroidi pour condenser les vapeurs de solvant et de co-solvant. Dans un bécher, on a dissous en chauffant légèrement 0,36 g de PtCl₂ dans 15 ml d'amine PRIMENE 81R, vendue par RHOM AND HAAS CO. On a ensuite ajouté 15 ml de produit SHELLSOL D70. Quand le chlorure de platine était totalement dissous, on a versé la phase liquide dans la colonne préalablement chauffée à 120 °C. On a alors injecté du chlorure d'hydrogène à un débit de 6,25 ml par seconde pendant 30 minutes de manière à transformer l'amine en chlorhydrate correspondant. Ensuite, conjointement au chlorure d'hydrogène, on a injecté un mélange constitué, sur une base molaire, de 25 % de méthylacétylène, de 13 % de propadiène, de 46 % de propylène, de 4 % de propane et de 12 % d'hydrocarbures en C₄ dans la colonne à un débit de 6,25 ml par seconde. La concentration en catalyseur dans le milieu liquide était de 45 mmol/l.

Les produits de la réaction obtenus au cours du temps ont été analysés par une analyse chromatographique en phase gazeuse en ligne. Les résultats sont présentés dans le tableau I ci-après. Dans ce tableau, le taux de conversion est le rapport entre la concentration initiale en méthylacétylène et en propadiène diminuée de sa concentration finale divisée par la concentration initiale, multiplié par 100 ; la sélectivité en 2-chloroprop-1-ène est le rapport entre la concentration finale en 2-chloroprop-1-ène divisée par la concentration initiale en méthylacétylène et en propadiène diminuée de sa concentration finale, multiplié par 100 ; le rendement en 2-chloroprop-1-ène est le rapport entre la concentration finale en 2-chloroprop-1-ène divisé par la concentration initiale en méthylacétylène et en propadiène multiplié par 100 ; le taux de conversion initial et la sélectivité initiale en 2-chloro-prop-1-ène rapportés au tableau 1, qualifiés sont les valeurs maximales observées après une mise en régime du réacteur d'au moins 10 heures. La stabilité du système catalytique est le temps nécessaire pour que le taux de conversion chute de 10 % par rapport à sa valeur maximale obtenue en début d'essai.

### Exemples 2-6

L'essai de l'exemple 1 a été répété avec d'autres composés du groupe VIIIa ou du groupe des lanthanides. Les résultats sont également présentés dans le tableau I ci-après.

**Tableau I**

| Exemple | Catalyseur | Température de réaction | taux de conversion initial (%) | sélectivité initiale en 2-chloroprop-1-ène (%) | stabilité (h) |
|---|---|---|---|---|---|
| 1 | PtCl₂ | 120 °C | 94 | 95 | 278 |
| 2 | PtBr₂ | 120 °C | 95 | 92 | 258 |
| 3 | acétylacétonate de Pt (II) | 120 °C | 97 | 87 | 330 |
| 4 | PtI₂ | 120 °C | 94 | 87 | 238 |
| 5 | H₂PtCl₆ | 150 °C | 98 | 84 | 133 |
| 6 | RhCl₃ | 150 °C | 44 | 87 | non mesuré |

### Exemples 7-8

On a répété les exemples 1 et 2 en présence de CuCl₂ comme co-catalyseur, avec un rapport molaire co-catalyseur/catalyseur égal à 5. Avec le système PtCl₂ - CuCl₂, on a obtenu un taux de conversion initial de 97 % et une sélectivité initiale en 2-chloroprop-1-ène de 92 %. La stabilité du système catalytique telle qu'exprimée ci-avant était de 410 heures. Avec le système PtBr₂ - CuCl₂, on a obtenu un taux de conversion initial de 98 % et une sélectivité initiale en 2-chloroprop-1-ène de 90 %. La stabilité du système catalytique telle que définie ci-avant était de 347 heures.

### Exemple 9

On a répété l'exemple 1 en utilisant du chlorure de méthyltrioctylammonium en tant que solvant. En effectuant la réaction à 140 °C, on a obtenu un taux de conversion initial de 91% et une sélectivité initiale en 2-chloroprop-1-ène de 93%. La stabilité du système catalytique telle que définie ci-avant était de 889 heures.

### Exemple 10

On a répété l'exemple 1 en utilisant de l'adiponitrile en tant que solvant. En effectuant la réaction à 150 °C, on a obtenu un taux de conversion initial de 40 % et une sélectivité initiale en 2-chloroprop-1-ène de 82 %.

### Exemple 11

On a répété l'exemple 1 en utilisant un mélange équivolumique d'adiponitrile et de primène 81 R en tant que solvant, en l'absence de co-solvant. En effectuant la réaction à 150 °C, on a obtenu un taux de conversion initial de 82 % et une sélectivité initiale en 2-chloroprop-1-ène de 84 %.

## Revendications

1. Procédé de préparation du 2-chloroprop-1-ène par réaction de méthylacétylène et/ou de propadiène avec du chlorure d'hydrogène dans un milieu liquide comprenant au moins
(a) un catalyseur d'hydrochloration comprenant au moins un composé choisi parmi les composés des métaux du groupe VIIIa et des lanthanides; et
(b) au moins un solvant organique capable de solubiliser le catalyseur.

2. Procédé selon la revendication 1 dans lequel le solvant est choisi parmi les nitriles, les composés organiques du phophore, les sels d'ammonium et leurs mélanges.

3. Procédé selon la revendication 1 ou 2 dans lequel le solvant est un sel d'ammonium de formule générale 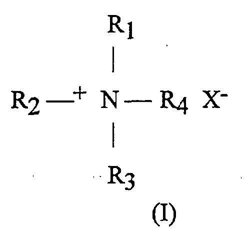 dans laquelle R₁, R₂, R₃ et R₄ représentent des atomes d'hydrogène, des groupements alkyle ou aryle, identiques ou différents, l'un d'eux au moins étant un groupement alkyle ou aryle et X représente un anion, de préférence l'anion chlorure;

4. Procédé selon la revendication 3 dans lequel le nombre d'atomes de carbone du sel d'ammonium est supérieur ou égal à 8 et inférieur ou égal à 40.

5. Procédé selon la revendication 4, dans lequel le sel d'ammonium est choisi parmi (a) un sel d'ammonium obtenu par la réaction du chlorure d'hydrogène avec les aminés primaires tert-alkyle PRIMENE® 81-R et PRIMENE® JM-T (b) le chlorure de méthyltrioctylammonium.

6. Procédé selon une quelconque des revendications 1 à 5 dans lequel le composé de métal du groupe VIIIa est un composé de platine, de rhodium ou de palladium.

7. Procédé selon une quelconque des revendications 1 à 6 dans lequel le composé de métal du groupe VIIIa est un composé de platine.

8. Procédé selon la revendication 7 dans lequel le composé de platine est un halogénure de platine (II).

9. Procédé selon l'une quelconque des revendications 1 à 8 dans lequel on met en oeuvre un cocatalyseur comprenant au moins un composé d'au moins un métal des groupes Ib ou IVb.

10. Procédé selon l'une quelconque des revendications 1 à 9 dans lequel le milieu liquide comprend en outre au moins un co-solvant organique.

11. Procédé se fabrication de 1,1,1,3,3-pentachlorobutane comprenant l'utilisation comme intermédiaire de synthèse du 2-chloropropène obtenu selon le procédé de l'une quelconque des revendications 1 à 10.

12. Procédé de fabrication de 1,1,1,3,3-pentafluorobutane (HFC-365mfc) comprenant l'utilisation comme précurseur de 1,1,1,3,3-pentachlorobutane obtenu selon le procédé de la revendication 11.

## Patentansprüche

1. Verfahren zur Herstellung von 2-Chlorprop-1-en durch Reaktion von Methylacetylen und/oder Propadien mit Chlorwasserstoff in einem flüssigen Medium, das wenigstens enthält
(a) einen Hydrochlorierungskatalysator, umfassend wenigstens eine Verbindung, die ausgewählt ist unter den Verbindungen der Metalle der Gruppe VIIIa und der Lanthaniden; und
(b) wenigstens ein organisches Lösungsmittel, das den Katalysator lösen kann.

2. Verfahren gemäß Anspruch 1, worin das Lösungsmittel ausgewählt ist unter den Nitrilen, den organischen Phosphorverbindungen, den Ammoniumsalzen und ihren Gemischen.

3. Verfahren gemäß Anspruch 1 oder 2, worin das Lösungsmittel ein Ammoniumsalz der allgemeinen Formel 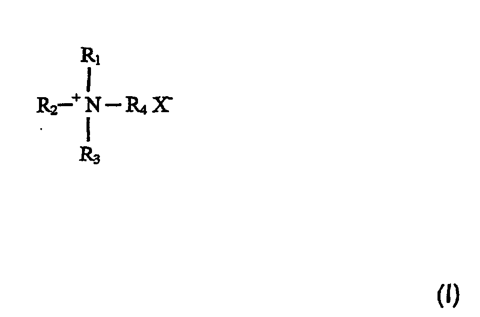 ist, worin R₁, R_{2,} R₃ und R₄ Wasserstoffatome, Alkyl- oder Arylgruppen, die gleich oder verschieden sind, darstellen, wobei wenigstens eines davon eine Alkyl- oder Arylgruppe ist, und X ein Anion darstellt, vorzugsweise das Chloridanion.

4. Verfahren gemäß Anspruch 3, worin die Anzahl an Kohlenstoffatomen des Ammoniumsalzes größer oder gleich 8 und kleiner oder gleich 40 ist.

5. Verfahren gemäß Anspruch 4, worin das Ammoniumsalz ausgewählt ist unter (a) einem Ammoniumsalz, das durch Reaktion von Chlorwasserstoff mit den primären tert.-Alkylaminen PRIMENE® 81-R und PRIMENE® JM-T erhalten wird, (b) Methyltrioctylammoniumchlorid.

6. Verfahren gemäß einem der Ansprüche 1 bis 5, worin die Verbindung eines Metalls der Gruppe VIIIa eine Platin-, Rhodium- oder Palladiumverbindung ist.

7. Verfahren gemäß einem der Ansprüche 1 bis 6, worin die Verbindung eines Metalls der Gruppe VIIIa eine Platinverbindung ist.

8. Verfahren gemäß Anspruch 7, worin die Platinverbindung ein Platin(II)-halogenid ist.

9. Verfahren gemäß einem der Ansprüche 1 bis 8, worin man einen Cokatalysator, der wenigstens eine Verbindung von wenigstens einem Metall der Gruppen Ib oder IVb umfasst, verwendet.

10. Verfahren gemäß einem der Ansprüche 1 bis 9, worin das flüssige Medium außerdem wenigstens ein organisches Hilfslösungsmittel umfasst.

11. Verfahren zur Herstellung von 1,1,1,3,3-Pentachlorbutan, umfassend die Verwendung von 2-Chlorpropen, erhalten gemäß dem Verfahren aus einem der Ansprüche 1 bis 10, als Synthesezwischenprodukt.

12. Verfahren zur Herstellung von 1,1,1,3,3-Pentafluorbutan (HFC-365mfc), umfassend die Verwendung von 1,1,1,3,3-Pentachlorbutan, erhalten gemäß dem Verfahren des Anspruchs 11, als Vorstufe.

## Claims

1. Process for the preparation of 2-chloro-1-propene by reaction of methylacetylene and/or propadiene with hydrogen chloride in a liquid medium containing at least
(a) a hydrochlorination catalyst which comprises at least one compound chosen from the compounds of the metals from group VIIIa and from the lanthanides; and
(b) an organic solvent capable of dissolving the catalyst.

2. Process according to Claim 1, in which the solvent is chosen from nitriles, organophosphorus compounds and ammonium salts, and mixtures thereof.

3. Process according to Claim 1 or 2, in which the solvent is an ammonium salt of general formula 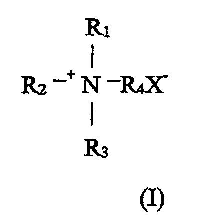 in which R₁, R₂, R₃ and R₄ represent hydrogen atoms, alkyl or aryl groups, which may be identical or different, at least one of them being an alkyl or aryl group and X represents an anion, preferably, the chloride anion.

4. Process according to Claim 3, in which the number of carbon atoms in the ammonium salt is greater than or equal to 8 and less than or equal to 40.

5. Process according to Claim 4, wherein the ammonium salt is chosen from (a) an ammonium salt obtained by reaction of hydrogen chloride with the tert-alkyl primary amines PRIMENE® 81-R and PRIMENE® JM-T (b) methyltrioctylammonium chloride.

6. Process according to any one of Claims 1 to 5, in which the compound of a metal from group VIII is a platinum, rhodium or palladium compound.

7. Process according to any one of Claims 1 to 6, in which the compound of a metal from group VIII is a platinum compound.

8. Process according to Claim 6, in which the platinum compound is a platinum(II) halide.

9. Process according to any one of Claims 1 to 8, in which a co-catalyst comprising at least one compound of at least one metal from groups Ib or IVb is used.

10. Process according to any one of Claims 1 to 9, in which the liquid medium also comprises at least one organic co-solvent.

11. Process for the manufacture of 1,1,1,3,3-pentachlorobutane comprising the use as synthesis intermediate of the 2-chloropropene obtained according to the process according to any one of claims 1 to 10.

12. Process for the manufacture of 1,1,1,3,3-pentafluorobutane (HFC-365mfc) comprising the use as precursor of 1,1,1,3,3-pentachlorobutane obtained according to the process of claim 11.
